(19) Europäisches Patentamt
European Patent Office
Office européen des brevets

(11) Numéro de publication: **0 004 222**
**B1**

(12) **FASCICULE DE BREVET EUROPEEN**

(45) Date de publication du fascicule de brevet: **28.10.81**

(21) Numéro de dépôt: **79400115.6**

(22) Date de dépôt: **26.02.79**

(51) Int. Cl.³: **C 07 D 317/72,**
C 07 C 49/493,
C 07 C 59/80, C 07 J 1/00

# (54) Nouveaux dérivés de la 4,5-séco estrane 3,5,17-trione, leur procédé de préparation ainsi que leur application à la préparation de composés thérapeutiquement actifs.

(30) Priorité: **06.03.78 FR 7806307**

(43) Date de publication de la demande:
**19.09.79 Bulletin 79/19**

(45) Mention de la délivrance du brevet:
**28.10.81 Bulletin 81/43**

(84) Etats Contractants Désignés:
**BE CH DE GB IT LU NL SE**

(56) Documents cités:
**CHEMICAL ABSTRACTS, vol. 79, no. 7, 20-8-1973**
**Columbus, Ohio, USA**
**A. TSUNEHIKO et al: "Total synthesis of 16-ethyl substituted steroids" page 381, 1ère colonne, abrégé no. 42732b**

**& YAKUGAKU ZASSHI 1973 93 (5) 566—9 (JAPAN)**

(73) Titulaire: **ROUSSEL-UCLAF**
**102, route de Noisy Boîte postale no.9**
**F-93230 Romainville (FR)**

(72) Inventeur: **Jolly, Jean**
**22 rue du Clos d'Orléans**
**F-94120-Fontenay S/Bois (FR)**
Inventeur: **Matra, Bernard**
**10 rue de Baligny**
**F-93600-Aulnay S/Bois (FR)**
Inventeur: **Rizzi, Primo**
**26 rue Circulaire**
**F-93250 Villemomble (FR)**

(74) Mandataire: **Tonnellier, Marie-José et al,**
**102, route de Noisy Boîte postale no 9**
**F-93230 Romainville (FR)**

**EP 0 004 222 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

Nouveaux dérivés de la 4,5-séco estrane 3,5,17-trione, leur procédé de préparation ainsi que leur application à la préparation de composés thérapeutiquement actifs

La présente invention concerne de nouveaux dérivés de la 4,5-seco estrane 3,5,17-trione, leur procédé de préparation, ainsi que leur application à la préparation de composés thérapeutiquement actifs.

L'invention a pour objet les composés de formule I:

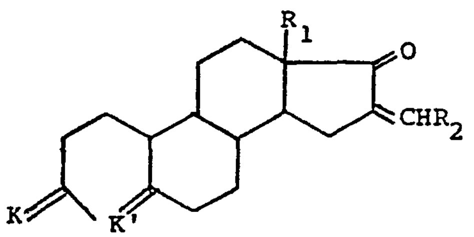

I

dans laquelle $R_1$ représente un radical alcoyle renfermant de 1 à 3 atomes de carbone et $R_2$ représente un atome d'hydrogène ou un radical alcoyle renfermant de 1 à 5 atomes de carbone, K et K', identiques ou différents, représentant une fonction cétone bloquée sous forme de cétal.

$R_1$ représente de préférence le radical méthyle ou éthyle. Lorsque $R_2$ représente un radical alcoyle, il s'agit de préférence du radical méthyle, éthyle, n-propyle, isopropyle, n-butyle, isobutyle ou ter-butyle. K et K' représentent de préférence un groupement alkylcétalcyclique ayant de 2 à 4 atomes de carbone et, notamment, l'éthylènecétal ou le propylènecétal ou bien un dialkylcétal comme le diméthylcétal ou le diéthylcétal.

L'invention a notamment pour objet les composés de formule I pour lesquels $R_1$ représente un radical méthyle, ceux pour lesquels R représente un radical méthyle ainsi que ceux pour lesquels K et K' représentent un groupement 1,2-éthanediyle acétalcyclique.

L'invention a plus particulièrement pour objet le 3,5-bis(1,2-éthanediyle acétalcyclique) 16-éthylidène 4,5-séco estrane 3,5,17-trione.

L'invention a également pour objet un procédé de préparation des composés de formule I tels que définis ci-dessus, caractérisé en ce que l'on soumet un composé de formule $II_A$

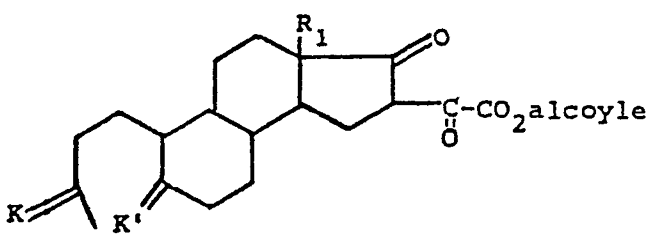

$II_A$

dans laquelle $R_1$, K et K' conservent la même signification que précédemment et alcoyle représente un radical alcoyle renfermant de 1 à 8 atomes de carbone, ou un enolate de métal alcalin de formule $II_B$

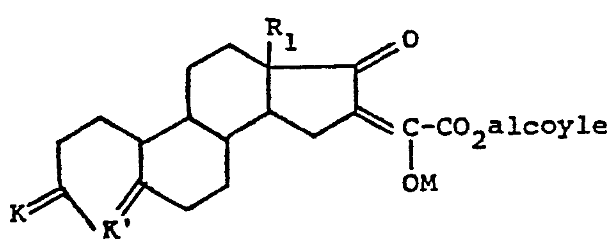

$II_B$

dans laquelle, $R_1$, K, K' et alcoyle conservent leur signification précédente et dans laquelle M représente un atome de métal alcalin, à l'action d'un aldéhyde de formule $R_2CHO$, dans laquelle $R_2$ conserve la même signification que précédemment, en présence d'une base, pour obtenir le composé de formule I correspondant:

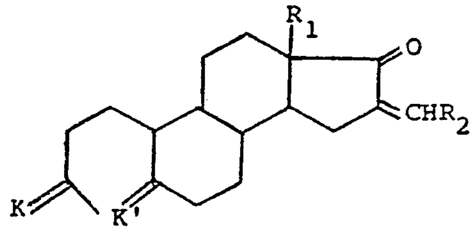

I

Dans un mode de réalisation préféré, M représente de préférence un atome de sodium ou de potassium et alcoyle représente de préférence un radical méthyle ou éthyle, la base utilisée est la soude, la potasse, un carbonate alcalin comme par exemple le carbonate de potassium on une amine tertiaire comme la triéthylamine.

Les composés de formules $II_A$ et $II_B$ qui sont nouveaux, peuvent être préparés en faisant réagir un oxalate d'alcoyle de formule:

$CO_2$alcoyle
|
$CO_2$alcoyle

dans laquelle alcoyle conserve sa signification précédente, en présence d'une base de métal alcalin M avec le composé de formule

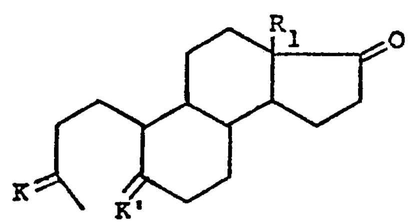

III

dans laquelle $R_1$, K et K' conservent la même signification que précédemment, pour obtenir le composé $II_B$ que l'on soumet, si désiré, à l'action d'un agent acide pour obtenir le composé de formule $II_A$. La base utilisée est de préférence la soude ou la potasse ou bien un alcoolate alcalin par exemple, le méthylate de sodium ou bien l'hydrure de sodium. L'agent acide utilisé est de préférence l'acide chlorhydrique ou l'acide acétique.

Les composés de formule III, précités, sont des produits connus que peuvent être préparés par exemple selon le procédé indiqué dans le brevet français 1490590.

L'invention a également pour objet l'application des composés de formule I caractérisée en ce que l'on soumet un composé de formule I

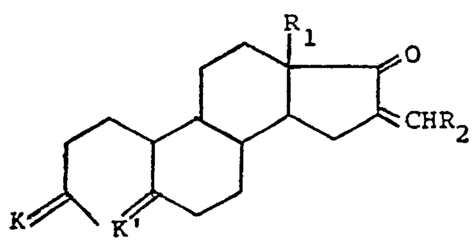

I

dans laquelle K, K', $R_1$ et $R_2$ conservent la même signification que précédemment, à l'action d'un agent d'hydrogénation capable de saturer la double liaison en 16, puis à celle d'un agent de réduction capable de réduire la fonction cétone en 17 et, enfin, à celle d'un agent d'hydrolyse acide pour obtenir le composé de formule

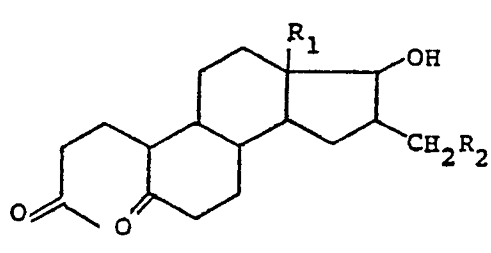

IV

que l'on soumet à l'action d'un agent basique pour obtenir le composé de formule V correspondant

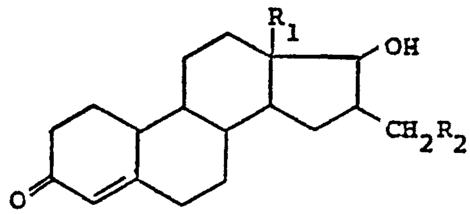

V

L'invention a notamment pour objet l'application des composés de formule I pour lesquels $R_2$ représente un radical méthyle, comme par exemple l'application de la 3,5bis(1,2-éthanediyl acétalcyclique) 16-éthylidène 4,5-seco estrane 3,5,17-trione à la préparation de la 16 β-éthyl 17 β-hydroxy-estr 4-ène-3-one.

L'agent d'hydrogènation capable d'hydrogéner la double liaison en 16 est, de préférence, l'hydrogène en présence du palladium.

l'agent de réduction capable de réduire la fonction cétone est de préférence, l'hydroborure de sodium ou l'hydrure mixte de lithium et d'aluminium.

L'agent d'hydrolyse acide est, de préférence, l'acide chlorhydrique, sulfurique, acétique, citrique ou paratoluènesulfonique.

L'agent basique au moyen duquel on effectue la cyclisation du cycle A est, notamment, un alcoolate alcalin tels que le méthylate de sodium, l'éthylate de sodium, le terbutylate de sodium ou de potassium, le teramylate de potassium ou encore une base alcaline telle que la soude ou la potasse. Les composés de formule V sont des produits connus; en général ils peuvent être préparés par exemple selon les procédés décrits par F.A. KINGL et Coll. dans STEROIDS 1959, p. 595 et suivantes, ou par HIRAGA et Coll. dans le brevet américain 3856829. Ces produits de formule V présentent d'intéressantes propriétés pharmacologiques. C'est ainsi, par exemple, que la 16 β-éthyl 17 β-hydroxy estr 4-ène 3-one, présente une très intéressante activité antiandrogène et trouve son utilisation dans le traitement de l'adénome de la prostate.

Les composés de l'invention sont donc nouveaux et leur procédé de préparation n'est pas évident. Le passage des composés $II_A$ ou $II_B$ aux composés I, en particulier, est original et n'est en rien suggéré par l'état de la technique.

Les composés de l'invention sont, de plus, d'un grand intérêt industriel, ils permettent en effet l'accès aux composés de formule V en cinq stades à partir des composés de formule III par un procédé simple et facilement industrialisable. Or, ces composés de formule III sont préparés facilement par synthèse totale et leur fabrication a atteint, depuis longtemps, le stade industriel car ils sont des intermédiaires de synthèse de très nombreux 19-nor stéroides vendus très largement dans le monde entier.

Les composés de l'invention permettent donc d'obtenir, par un procédé simple et facilement industrialisable, les produits de formule V obtenus, jusqu'ici, par un procédé long et difficilement industrialisable, en neuf stades à partir de l'oestrone par exemple, selon les procédés décrits dans Z PHYSIOL Chem. 1932, *208*, 127, J. CHEM Soc. *1938* 1997, brevet français 4957M et brevet américain 3856829.

Les exemples suivants illustrent l'invention sans toutefois la limiter.

### Exemple 1

*3,5-bis (1,2-éthanediyl acétalcyclique)16-éthylidène 4,5-seco estrane 3,5,17-trione.*

Stade A: 3,5-bis(1,2-éthanediyl acétalcyclique)3,5, 17,α tétraoxo 4,5-séco estrane 16 β-acétate d'éthyle.

On introduit 200 g de 3,5-bis(1,2-éthanediyl acétalcyclique) 4,5-seco estrane 3,5, 17-trione (préparé selon le procédé décrit dans le brevet français 1490590), dans 600 $cm^3$ de diméthylformamide. On introduit dans la solution ainsi obtenue à −10° ±2°C., 66 g de méthylate de sodium puis à 0° ± 2°C. 160 $cm^3$ d'oxalate d'éthyle. On agite la solution obtenue pendant 2 heures à 0°, + 5°C et ajoute 100 $cm^3$ d'acide acétique. On amorce la cristallisation de la solution ainsi obtenue par grattage dans un mélange d'eau et de glace 50—50. On essore, lave à l'eau la suspension obtenue et sèche. On obtient ainsi 242,5 g du produit recherché fondant à environ 100°C. que l'on utilise tel quel dans le stade suivant.

Stade B: 3,5-bis(1,2-éthanediyl acétalcyclique) 16-éthylidène 4,5-seco estrane 3,5,17-trione.

On introduit sous agitation et sous courant d'azote à 20°C., 242,5 g du produit préparé au stade A dans 243 $cm^3$ de méthanol anhydre, puis on ajoute dans la suspension ainsi obtenue 121 g de carbonate de potassium. On agite la suspension obtenue pendant 15 minutes à température ambiante, amène à −5° ± 2°C et ajoute 121 $cm^3$ d'acétaldéhyde. On agite à nouveau la suspension obtenue pendant 2 heures à 0°C., laisse remonter la température à 20°C. et agite pendant 16 heures à cette température. On amorce la cristallisation par grattage dans un mélange de 182 g de phosphate monopotassique et de 730 $cm^3$ d'un melange d'eau et de glace 50—50. On essore et lave le précipité obtenu à l'eau. On

dissout le produit obtenu dans 1200 $cm^3$ de chlorure de méthylène. On décante la phase aqueuse et sèche la solution organique sur sulfate de magnésium. On essore et rince au chlorure de méthylène. On introduit dans la solution chlorométhylènique, sous agitation à 20° ± 2°C. et en trente minutes, 242 g d'alumine hydratée. On maintient l'agitation pendant une heure, on essore et rince avec 240 $cm^3$ de chlorure de méthylène. On concentre la solution organique jusqu'à l'obtention d'un volume de 240 $cm^3$. On reprend sous agitation et au reflux par 1200 $cm^3$ d'éther isopropylique. Il y a dissolution totale. On concentre la solution ainsi obtenue jusqu'à l'obtention d'un volume de 600 $cm^3$. On refroidit la suspension obtenue à —10°C. et la maintient sous agitation pendant une heure à cette température. On essore la précipité obtenu et le rince avec 120 $cm^3$ d'éther isopropylique. On sèche le produit obtenu et obtient ainsi 156,5 g du produit recherché fondant à 131°C.

Exemple 2

*Application du composé de l'exemple 1 à la préparation de la 16 β*-éthyl 17 β-hydroxy estr 4-ène 3-one.

Stade A: 16 β-éthyl 17-β-hydroxy 4,5-seco estrane 3,5-dione

a) *Hydrogénation de la double liaison en 16*

On introduit dans 400 $cm^3$ de méthanol, 50 g du produit de l'exemple 1, 0,4 $cm^3$ de pyridine, 1,250 g de charbon actif à 10% de palladium. On fait passer un courant d'hydrogène. Après absorption de 1700 $cm^3$ d'hydrogène, il y a dissolution totale. Après la fin de l'absorption, on laisse deux heures sous agitation. On essore le catalyseur, le rince au méthanol et réunit les phases méthanoliques.

b) *Réduction de la fonction cétone*

On introduit 50 $cm^3$ d'eau et 15 $cm^3$ de soude normale dans 500 $cm^3$ de la solution méthanolique obtenue au paragraphe a). On introduit ensuite, en une heure, 2,45 g d'hydroborure de sodium, maintient la solution ainsi obtenue pendant 15 à 20 heures sous agitation. On concentre la solution jusqu'à l'obtention d'un volume de 250 $cm^3$. On introduit, peu à peu, sous agitation et à 20°C. 5 $cm^3$ d'acide acétique et laisse sous agitation pendant 15 minutes, à 20°C. On verse lentement la solution obtenue dans 2.500 $cm^3$ d'un mélange d'eau et de glace (50—50), la maintient sous agitation pendant 30 minutes. On essore le précipité obtenu, le lave à l'eau et l'essore à nouveau. On obtient 107,5 g de produit que l'on utilise tel quel pour l'opération suivante.

c) *Hydrolyse des fonctions cétal*

On introduit les 107,5 g de produit obtenu au paragraphe b) dans un solution renfermant 150 $cm^3$ d'acide acétique et 93 $cm^3$ d'eau déminéralisée. On porte la suspension obtenue à 40°C. sous agitation et la maintient pendant 2 heures dans ces conditions. On refroidit ensuite la suspension à 20°C. et la verse dans 1.000 $cm^3$ d'un mélange d'eau et de glace (50—50). On maintient la suspension sous agitation pendant 30 minutes, essore la précipité obtenu, le lave à l'eau et le sèche. On obtient ainsi 34,4 g de produit brute que l'on purifie par recristallisation dans le toluène. On obtient ainsi 29,340 g de produit recherché, fondant à 113°C.

*Stade B:* β-éthyl 17 β-hydroxy estr 4-ène 3-one

On introduit 25 g du produit préparé au stade A dans 250 $cm^3$ de méthanol. On chauffe à 50°C. sous agitation la suspension obtenue et ajoute 9,4 $cm^3$ d'une solution de lessive de soude à 400 g par litre. On maintient la solution ainsi obtenue à 50°C. pendant 30 minutes. On ajoute ensuite à 50°C. un mélange de 79 $cm^3$ d'eau et 5,8 $cm^3$ d'une solution d'acide acétique à 110%. On refroidit la suspension à 0° + 5°C. sous agitation et maintient 30 minutes dans ces conditions. On essore, lave au méthanol et à l'eau, puis sèche le précipité obtenu. On obtient ainsi 21,4 g de produit brut que l'on purifie par recristallisation dans l'acétate d'éthyle. On obtient ainsi 19,800 g du produit recherché, fondant à 152°C—153°C.

**Revendications**

1. Les composés de formule I

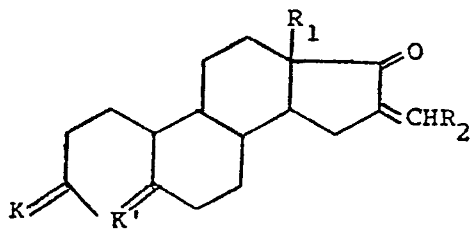

I

dans laquelle $R_1$ représente un radical alcoyle renfermant de 1 à 3 atomes de carbone et $R_2$ représente

un atome d'hydrogène ou un radical alcoyle renfermant de 1 à 5 atomes de carbone, K et K', identiques ou différents, représentant une fonction cétone bloquée sous forme de cétal.

2. Les composés de formule I, tels que définis à la revendications 1, pour lesquels $R_1$ représente un radical méthyle.

3. Les composés de formule I, tels que définis à la revendication 1 ou 2, pour lesquels $R_2$ représente un radical méthyle.

4. Les composés de formule I tels que définis à la revendication 1, 2 ou 3, pour lesquels K et K' représentent un groupement 1,2-éthanediyle acétalcyclique.

5. Le 3,5-bis(1,2 éthanediyle acétalcyclique) 16-éthylidène 4,5- seco estrane 3,5,17-trione.

6. Procédé de préparation des composés de formule I, tels que définis à la revendication 1, caractérisé en ce que l'on soumet un composé de formule $II_A$

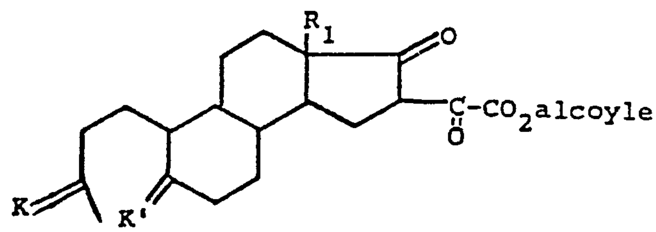

$II_A$

dans laquelle $R_1$, K et K' conservent la même signification que dans la revendication 1 et alcoyle représente un radical alcoyle renfermant de 1 à 8 atomes de carbone, ou un énolate de métal alcalin de formule $II_B$

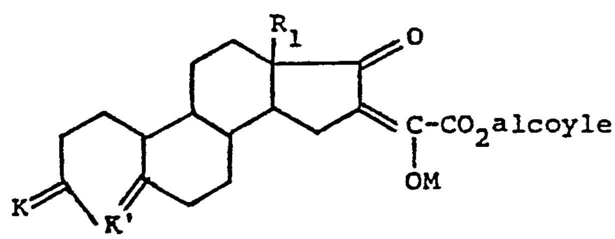

$II_B$

dans laquelle $R_1$, K, K' et alcoyle conservant leur signification précédente et dans laquelle M représente un atome de métal alcalin, à l'action d'un aldéhyde de formule $R_2CHO$, dans laquelle $R_2$ conserve la même signification que dans la revendication 1, en présence d'une base, pour obtenir le composé de formule I correspondant:

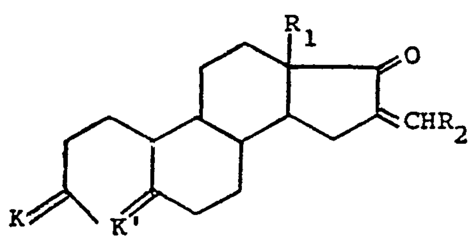

I

7. Application des composés de formule I, caractérisé en ce que l'on soumet un composé de formule I

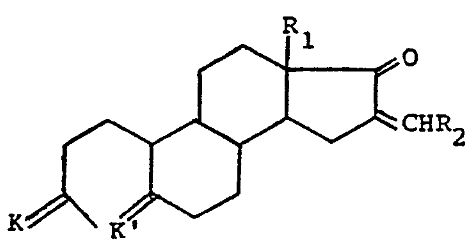

I

dans laquelle K, K', $R_1$ et $R_2$ conservent la même signification que dans la revendication 1, à l'action d'un agent d'hydrogénation, capable de saturer la double liaison en 16, puis à celle d'un agent de

réduction capable de réduire la fonction cétone en 17 et, enfin, à celle d'un agent d'hydrolyse acide pour obtenir le composé de formule

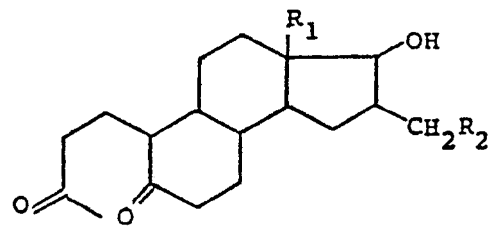

IV

que l'on soumet à l'action d'un agent basique pour obtenir le composé de formule V correspondant

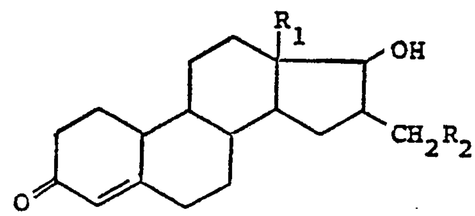

V

8. Application selon la revendication 7 caractérisée en ce que $R_2$ représente un radical méthyle.

9. Application selon la revendication 7 de la 3,5-bis(1,2-éthanediyl acétal cyclique) 16-éthylidène 4,5-seco estrane 3,5,17-trione à la préparation de la 16$\beta$-éthyl 17$\beta$-hydroxy-estr-4-ène-3-one.

## Claims

1. The compounds of formula I

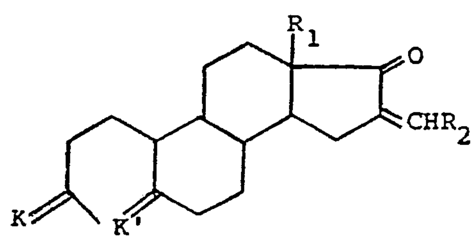

I

in which $R_1$ represents an alkyl radical containing from 1 to 3 carbon atoms and $R_2$ represents a hydrogen atom or an alkyl radical containing from 1 to 5 carbon atoms, K and K', being the same or different, representing a blocked ketone function in the form of a ketal.

2. The compounds of formula I, as defined in Claim 1, for which $R_1$ represents a methyl radical.

3. The compounds of formula I, as defined in Claim 1 or 2, for which $R_2$ represents a methyl radical.

4. The compounds of formula I, as defined in Claim 1, 2 or 3, for which K and K' represent a cyclic 1,2-ethanediyl acetyl group.

5. 3,5-bis(cyclic 1,2-ethanediyl acetal) 16-ethylidene 4,5-seco estrane 3,5,17-trione.

6. Process for preparing the compounds of formula I, as defined in Claim 1, characterised in that a compound of formula $II_A$

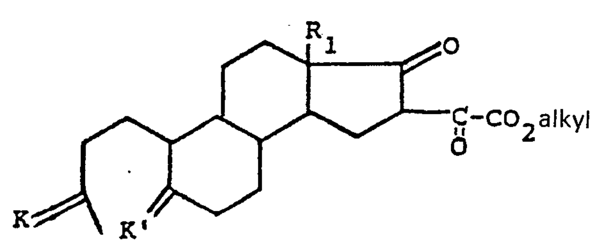

$II_A$

in which $R_1$, K and K′ keep the same meaning as in Claim 1 and alkyl represents an alkyl radical containing from 1 to 8 carbon atoms, or an alkali metal enolate of formula $II_B$

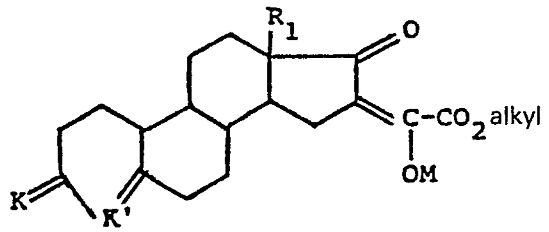

$II_B$

in which $R_1$, K, K′ and alkyl keep their above meaning and in which M represents an alkali metal atom, is subjected to the action of an aldehyde of formula $R_2CHO$, in which $R_2$ keeps the same meaning as in Claim 1, in the presence of a base, to obtain the corresponding compound of formula I:

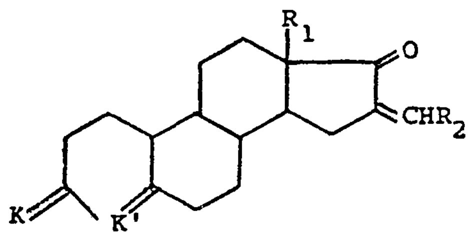

I

7. Use of the compounds of formula I, characterised in that a compound of formula I

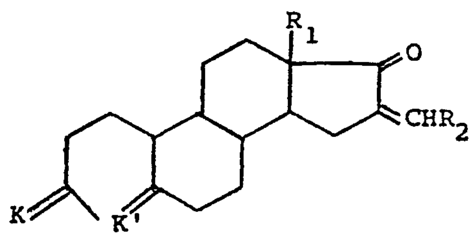

I

in which K, K′, $R_1$ and $R_2$ keep the same meaning as in Claim 1 is subjected to the action of a hydrogenation agent capable of saturating the double bond at 16, then to that of a reducing agent capable of reducing the ketone function at 17 and finally to that of an acid hydrolysis agent to obtain the compound of formula

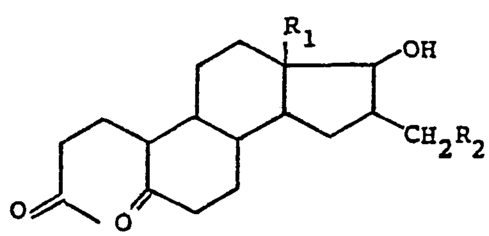

IV

which is subjected to the action of a basic agent to obtain the corresponding compound of formula V

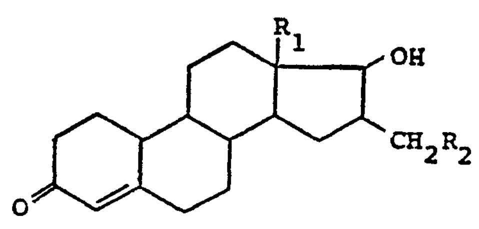

V

8. Use according to Claim 7, characterised in that $R_2$ represents a methyl radical.

9. Use according to Claim 7 of 3,5-bis (cyclic 1,2-ethanediyl acetal) 16-ethylidene 4,5-seco estrane 3,5,17-trione in the preparation of 16β-ethyl 17β - hydroxy - estr - 4 - ene - 3 - one.

## Patentansprüche

1. Verbindungen der Formel I

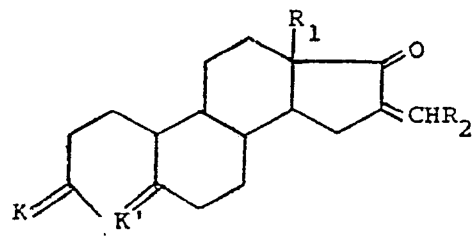

I

worin $R_1$ einen Alkylrest mit 1 bis 3 Kohlenstoffatomen bedeutet und $R_2$ ein Wasserstoffatom oder einen Alkylrest mit 1 bis 5 Kohlenstoffatomen bedeutet und K und K', die gleich oder verschieden sein können, eine in Form des Ketals blockierte Ketonfunktion darstellen.

2. Verbindungen der Formel I gemäß Anspruch 1, worin $R_1$ einen Methylrest bedeutet.

3. Verbindungen der Formel I gemäß Anspruch 1 oder 2, worin $R_2$ einen Methylrest bedeutet.

4. Verbindungen der Formel I gemäß Anspruch 1, 2 oder 3 worin K und K' eine 1,2-Äthandiylcyclischacetalgruppe darstellen.

5. 3,5-Bis-(1,2-äthandiyl-cyclisch-acetal)-16-äthyliden-4,5-seco-östran-3,5,17-trion.

6. Verfahren zur Herstellung von Verbindungen der Formel I gemäß Anspruch 1, dadurch gekennzeicnet, daß man eine Verbindung der Formel $II_A$,

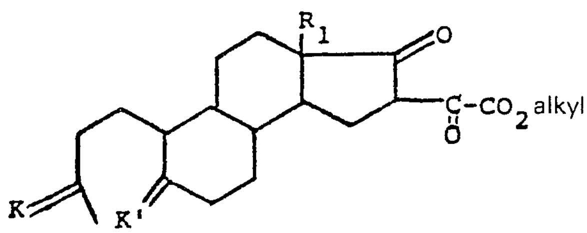

$II_A$

worin $R_1$. K und K' die in Anspruch 1 angegebene Bedeutung besitzen und Alkyl einen Alkylrest mit 1 bis 8 Kohlenstoffatomen bedeutet, oder ein Alkalienolat der Formel $II_B$,

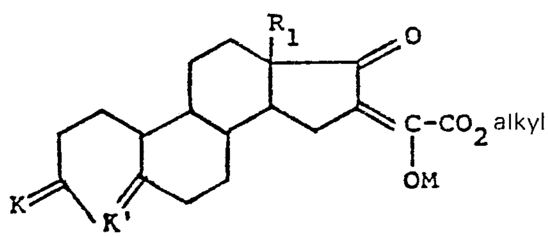

$II_B$

worin $R_1$, K, K' und Alkyl die vorstehend angegebene Bedeutung besitzen und worin M ein Alkalimetall bedeutet, der Einwirkung eines Aldehyds der Formel $R_2CHO$, worin $R_2$ die in Anspruch 1 angegebene Bedeutung besitzt, in Gegenwart einer Base unterzieht, um die entsprechende Verbindung der Formel I

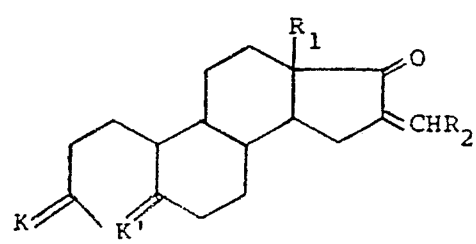

I

zu erhalten.

7. Verwendung der Verbindungen der Formel I, dadurch gekennzeichnet, daß man eine Verbindung der Formel I,

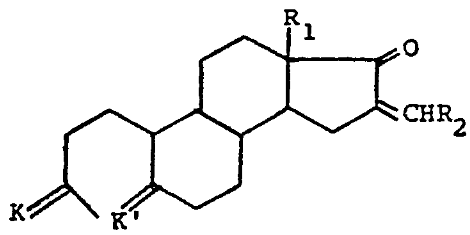

I

worin K, K', $R_1$ und $R_2$ die in Anspruch 1 angegebene Bedeutung besitzen, der Einwirkung eines Hydrierungsmittels, das in der Lage ist, die Doppelbindung in 16-Stellung zu sättigen, und danach derjenigen eines Reduktionsmittels das in der Lage ist, die Ketonfunktion in 17-Stellung zu reduzieren, und schließlich derjenigen eines sauren Hydrolysemittels unterzieht, um die Verbindung der Formel IV

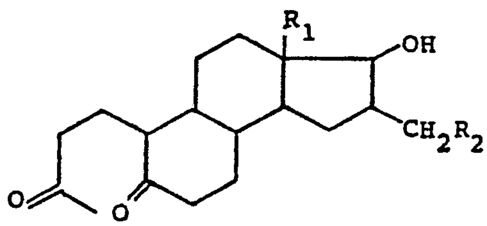

IV

zu erhalten, die man der Einwirkung eines basischen Mittels unterzieht, um die entsprechende Verbindung der Formel V

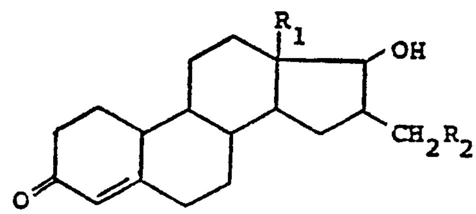

V

zu erhalten.

8. Verwendung gemäß Anspruch 7, dadurch gekennzeichnet, daß $R_2$ einen Methylrest bedeutet.

9. Verwendung gemäß Anspruch 7 von 3,5 - Bis - (1,2 - äthandiyl - cyclisch - acetal) - 16 - äthyliden - 4,5 - seco - östran - 3,5,17 - trion zur Herstellung von 16$\beta$ - Äthyl - 17$\beta$ - hydroxy - östr - 4 - en - 3 - on.